(19) **Europäisches Patentamt / European Patent Office / Office européen des brevets**

(11) **EP 1 257 236 B1**

(12) **EUROPEAN PATENT SPECIFICATION**

(45) Date of publication and mention of the grant of the patent:
**30.05.2007 Bulletin 2007/22**

(21) Application number: **01923554.8**

(22) Date of filing: **24.01.2001**

(51) Int Cl.:
**A61F 2/16** (2006.01)

(86) International application number:
**PCT/EP2001/000734**

(87) International publication number:
**WO 2001/062188 (30.08.2001 Gazette 2001/35)**

(54) **INTRAOCULAR LENSES**

INTRAOKULARE LINSE

LENTILLES INTRAOCULAIRES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **24.02.2000 SE 0000611**

(43) Date of publication of application:
**20.11.2002 Bulletin 2002/47**

(73) Proprietor: **AMO Groningen B.V.**
**9700 AX Groningen (NL)**

(72) Inventors:
• **BOGAERT, Theo, T., M.**
**NL-9742 HS Groningen (NL)**
• **MEIJER, Sieger, T.**
**NL-9471 RE Zuidlaren (NL)**

(74) Representative: **Holmberg, Martin Tor**
**Bergenstrahle & Lindvall AB**
**P.O. Box 17704**
**118 93 Stockholm (SE)**

(56) References cited:
**WO-A-98/17205**      **US-A- 5 258 025**
**US-A- 5 776 192**      **US-A- 5 913 898**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

**Description of invention**

The present invention refers to implantable phakic intraocular lenses (IOLs) suitable as correction lenses together with the intact natural crystalline lens. The inventive lenses a provided with a posterior surface, which admits a more anatomical fit in the posterior chamber of the eye, thereby minimizing the risks of disturbing the natural lens.

**Background of the invention**

As a consequence of that surgical procedures in the eye when replacing a defect natural lens have been more perfected and less traumatic, for example after the development of resilient lenses capable of being folded through a small incision in the eye, phakic intraocular lenses are increasingly conceivable as an alternative to correct for optical deficiencies besides spectacles and conventional contact lenses.

In a general sense phakic lenses can be considered for implantation, either in the anterior (front) chamber of the eye between the cornea and the iris, or in the posterior (rear) chamber located between the iris and the natural crystalline lens.

Phakic lenses positioned in the anterior chamber have been considered as desirable in several earlier embodiments for the reason that this chamber is considerably larger than the posterior chamber and thereby admitting a less complicated surgical process. However, these types of lenses show series of drawbacks essentially related with an irritation action from the support means (haptics) on the sensitive eye structures. For example, the support means can, when positioned in the corner between cornea and iris, disturb the aqueous outflow and consequently generate an increase in the intraocular pressure, a condition, which at worst may induce glaucoma. Pressure on the support means on the anterior chamber angle can disturb blood circulation and cause pupil ovalization. Further, the support means and the optic may come in contact with the corneal endothelium and cause endothelial dystrophy with resulting loss of corneal transparency. Alternatively, the support means have been suggested to be fixated directly to the iris by various attachment means. A negative consequence of iris fixation is irritation of the iris.

It is a general complication when designing IOLs to be implanted in the posterior chamber between the iris and the natural crystalline lens that the available space is small. Consequently, the lenses cannot be bulky as frequently is required when a high power optical correction is considered. In particular, consideration must be taken to avoid or restrict any contacts with the intact natural crystalline lens, in order to prevent it from damages, which may lead to local opacifications, or at worst case cataract formation.

Considerations must also be taken to that contact with iris could result in abrasive damages with resulting pigment dispersion and that the pupil must not be blocked, so the flow of aqueous humor is prevented which may lead to raised intraocular pressure and reduced circulation of nutrients and metabolites to and from the natural crystalline lens.

US Patent 4,585,456 (Blackmore) discloses an early version of a posterior chamber phakic IOL that is fixed in position by appendages that contact the ciliary sulcus. In the embodiments described herein no considerations have been taken to minimize contact with surrounding sensitive eye structures.

US Patent 4,769,035 (Kelman) also describes a method of correcting eyesight with an IOL positioned between the iris and the natural lens. The method involves a step of determining the shape of the natural lens in its flattest natural condition (i.e. non-accommodated) and forming a posterior surface of the IOL which has a curvature conforming with the natural lens in said condition. The resulting IOLs will be in permanent contact with the natural lens, which means that there will be a risk that damages are caused in its structure with cataract formation as a consequence.

To minimize the risk of contacting the natural lens several designs have been suggested where the lens is provided with supporting means to secure to the ciliary sulcus. US Patent 5,258,025 (Fedorov) relates to a correction IOL for implantation between the iris and the natural lens having modified supporting elements with a tapered peripheral part serving to protect the ocular tissues from such interaction with the lens that could result in postoperative inflammations.

European Patent 0 563 602 (Chiron Adatomed) discloses a correction lens for the posterior chamber of the eye provided with an outer haptic portion designed so as to distance the correction lens from the natural lens while enabling a securing contact with the zonule fibers of the ciliary sulcus in order to properly define the lens position. The geometric configuration of the correction lens and the presence of openings for fluid circulation in the haptics serve to provide for that the anterior surface of the natural lens remains accessible for the metabolic processes occurring at this location.

Fixating correction lenses in the ciliary sulcus is not preferred, because the sulcus has an irregular shape varying between different individuals and it will be difficult to accurately size the overall lens length. It will either be to short, or have an unpredictable central distance to the natural lens, which means that the optical correction cannot be sufficiently controlled. It is another disadvantage that the correction lens is in a fixed in a predetermined position when the pupil is off-center. Further, the force of the sulcus-fixated lens exerted on the ciliary tissues increases the risk of a blood/aqueous barrier break.

It has therefore been suggested to design correction lenses for the posterior chamber to float on a layer of aqueous humor in front of the natural lens, without having any permanent securing contact with the ciliary sulcus periphery and follow the movements of the pupil. The length of a free floating lens is smaller than the sulcus diameter and the lens will rest on the zonula or be pushed forwards by the aqueous humor secreted by the ciliary body. The aqueous flow can thereby more easily reach the entire surface of the lens and bring nutrients to the natural lens surface and remove derivatives from its metabolic process.

International Patent Applications published as WO 89/02252 (Mezhotraslevol Nauchno-Tekhnichesky Komplex "Microkhirurgia Glaza") and WO 95/15733 (Voir Et Vivre) disclose floating corrective IOLs for the posterior chamber wherein the supporting elements (haptic part) of the lens are considerably smaller in diameter than the ciliary sulcus and the lenses are secured in a corrected position by iris contact from dilations of the pupil acting on the optical part which protrudes into the anterior chamber of the eye. These lenses suffer from the drawback from that their optical parts have a limited diameter and that the edge of the optical part can scatter light with the result of undesired formation of halo-shaped optical sensations.

US Patent 5,480,428 (Fedorov) discloses a floating posterior chamber correction lens with a protruding optical part centered by the iris. In order to enable fluid circulation, in case the pupil is blocked by the lens, the optical part is provided with a central hole thereby avoiding the need of creating a hole in the iris (iridotomy). Further, this lens has a positioning element with a posterior surface having a radius largely following that of the natural lens, which is larger than the radius of the posterior surface of the optical part. This design means that the lens will be bulky, especially if a high refractive outcome is desired, so that the lens may be pushed forwards and apply a force against the iris by the pressure exerted from accommodation of the natural lens. Permanent disturbances of the iris can at worst induce pigment dispersion followed by pigmentary glaucoma. There is also a risk that a permanent forward dislocation of the iris can lead to that the anterior chamber angle closes the trabecular meshwork involved in aqueous humor transportation. When this condition is permanent the intraocular pressure can be chronically raised.

US Patent No. 5,913,898 (Feingold) discloses a corrective lens for the posterior chamber of the eye provided with features on its anterior side that enables a more smooth contact with the iris so as to avoid damages of wear due to long term contact. This patent also discloses means for allowing sufficient circulation of eye fluids around the lens, so as to avoid the built up of pressure differences between the posterior and the anterior chambers of the eye.

WO 98/17205 (IVI) discloses a further developed floating corrective lens for the posterior chamber wherein more considerations have been taken to the interaction with iris by making the lens thinner and having the optic part substantially in the posterior chamber. However, this lens design cannot avoid pupillary block of circulating fluids, so the need of applying iridotomies (holes in the iris) remains. Further, these lenses do not consider any precautionary means to avoid damages to the zonulas and to surface of the natural lens. This document discloses the features of the preamble of claim 1.

Even if efforts are made in the corrective lenses for the posterior chamber to avoid disturbing the highly sensitive natural lens and thereby risk to induce the formation of opaque areas or a cataract, there still is a need to develop a lens having a posterior surface that contributes to avoid or decrease the risk of inflicting damages to the natural lens or to the zonulas. As will become apparent in the below in the descriptive part and the objects of invention, the presently inventive lens aims to provide a solution to these problems.

It is an object of the present invention to provide a corrective lens having an improved adaptation to the free space available in the posterior chamber of the eye between the iris and the natural lens so as to avoid any disturbances to the natural tissues.

It is another object of the present invention to provide a corrective lens for the posterior chamber of the eye that minimizes the contacts with the natural lens so as to avoid the cataract formation.

It is a further object of the present invention to provide a corrective lens having a posterior surface facing the natural lens designed to avoid stress concentration in this region.

It is a still further object of the present invention to provide a corrective lens with supporting means, which are adapted so as to avoid inflicting damages to the zonulas or to the ciliary sulcus.

These and other objects of the invention, which will be described in more detail below, or will be obvious from later explanations, are met with the presently invented lenses as disclosed in the following descriptive sections.

## Description of invention

In the most general terms, the present invention pertains to an intraocular correction lens for implantation in the posterior chamber of the eye between the iris and the intact natural lens. The correction lens comprises a centrally located optical part capable of providing an optical correction and a peripherally located supporting element capable of maintaining said optical part in the central location. If regarded from above, the correction lens will generally have total length from about 9 to about 13 mm and a width from about 6 to about 8 mm which values are confined by the size of the posterior chamber of the individual patient. In accordance with the present invention, the optical part and the support element together have a concave posterior surface, which after implantation will face the natural lens. The concave posterior surface is a part of a non-spherical surface that is rotation symmetric around the optical axis of said optical part. The intersection between the non-spherical surface and any plane containing the optical axis represents a flawless curve free from discontinuities and points of inflection. A representative flawless curve is formed by the intersection of a plane containing both the optical axis and the longest symmetry axis of the lens contour and said non-spherical surface.

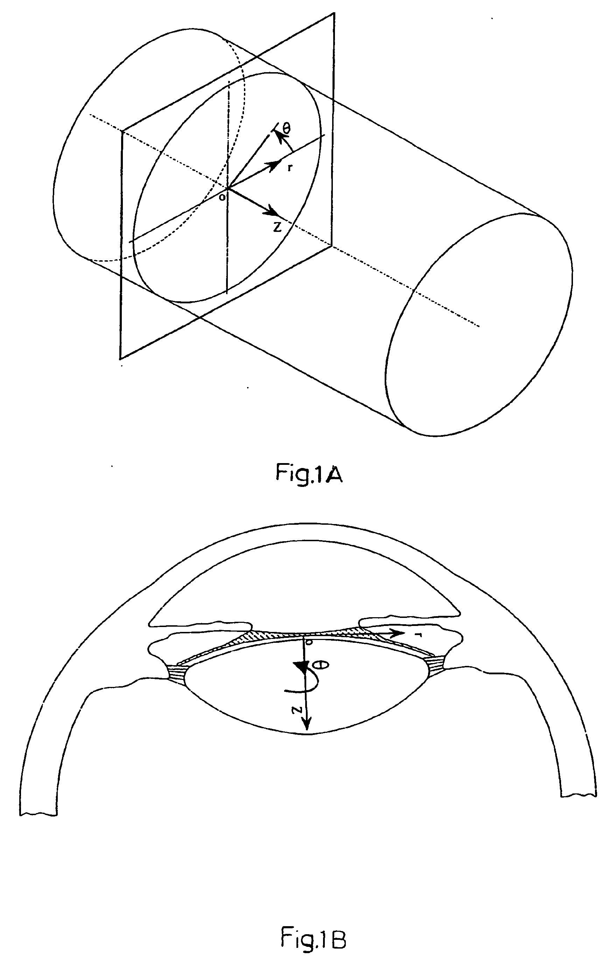

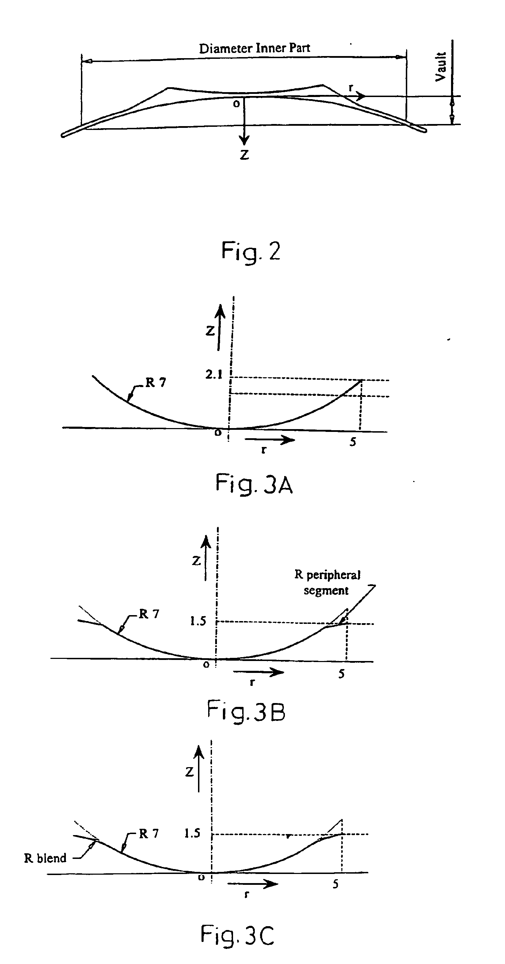

In the context of the present invention, a flawless curve free from discontinuities is defined by that the curve is expressed by a continuous mathematical function. Further, that the curve is defined to be free from points of inflection is defined herein as the mathematical function defining, or approximating, the curve should not have any points of inflection, i.e. the second derivative of the mathematical function is not equal to zero at any point of the curve. In order to conceive the curves, a contemplated cylindrical coordinate system is introduced having a z-axis coinciding with the optical axis and radius r/ angle $\theta$ coordinates in a plane perpendicular to the optical axis and the origin of vertex of the correction lens. In the outlined coordinate system the above-mentioned flawless curve will be represented by a mathematical function $z = f(r, \theta)$, wherein r has positive value, having an extreme point on the z-axis (optical axis) and no points of inflection. The coordinate system is demonstrated in Fig. 1 and 2 in the detailed part of the description.

According to one aspect of the invention, the posterior flawless curve of the correction lens is at least extended in a direction towards the lens periphery within an area defined by the projection of the natural lens on the posterior surface of said correction lens in a direction parallel to the optical axis. According to another aspect of the invnetion the posterior flawless curve of the lens has substantially the same extension as the width of the lens, i.e. a total length of from about 6 to 8 mm symmetrically distributed around the optical axis.

The natural lens typically varies in diameter between about 9 and 10.5 mm, depending on the individual patient and his/her age. The diameter of the natural lens can be estimated as a part of the pre-surgical considerations and a suitable correction lens with a suitably extended flawless curve can thereby readily be selected. The flawless curve should at least be extended in a direction towards the lens periphery within an area defined by the projection of the zonula-free natural lens on the posterior surface of said correction lens in a direction parallel to the optical axis. Zonula free is defined herein, as the part of the lens that is substantially free from zonulas attached to the lens, through which the state of accommodation of the lens is regulated by the ciliary muscles. The extension of the flawless curve sufficiently covers the natural lens, thereby providing for that no local pressure points are built up that can form stress concentration points or zones on the natural lens which may impair its natural metabolism and form local opacifications which in worst case result in cataract formation and the subsequent need of surgical intervention.

The support elements comprise an inner part neighboring the optical part and an outer, peripheral part, which is designed to at least partially to be in contact with the ciliary sulcus and the zonulas. According to a first embodiment, the peripheral part is flawlessly connected to the inner part of the support elements. The flawless curve as defined above will thereby continue and extend in a peripheral curve which is defined as the intersection between a plane containing the optical axis and the peripheral part of the posterior non-spherical surface. According to another embodiment, the peripheral part of the support elements is connected to the inner part at a point of inflection of the curve represented by the intersection of the non-spherical posterior surface of correction lens and a plane containing the optical axis.

In accordance with a preferred embodiment, the peripheral part of the support means follows a curve that converges towards a plane perpendicular to the optical axis. This ensures that the support means are directed from the zonulas attached to the natural lens and that the lens advantageously adapts to be accommodated in the free space confined by the posterior chamber of the eye between the iris and the natural lens.

When the posterior surface is designed according to the invention, it is highly preferred that the central radius of the posterior concave surface of the optical part is different than the central radius of natural lens in its non-accommodated state. In this context, the central radius (of the posterior surface) is defined as the radius in the near proximity of the intersection of the optical axis and the correction lens and the natural lens, respectively. Accordingly, by selecting different radii, the risk of adherence between the implanted lens and the natural lens is avoided. In a first embodiment, the central radius of the posterior surface is substantially smaller than the central radius of the natural lens. Preferably, the central radius of the posterior surface is less than about 8 mm and more preferably less than about 7 mm. This embodiment is suitable for young patients less than about 30 years. In a second embodiment, especially suitable for patients older than about 30 years, the central radius of the posterior surface of the correction lens is substantially larger than the central radius of the natural lens. Preferably, the central radius of the posterior surface of the correction lens is larger than about 12 mm and more preferably larger than about 14 mm.

According to a specific embodiment, the radius of the posterior surface increases from the central part proximal to the optical axis towards the periphery. In this embodiment, at a point proximal to the periphery of the optical part the radius will be larger than at a central point proximal to the optical axis.

When designing the lenses of the present invnetion, the central radius of its posterior surface is determined with respect to an estimated value of the radius of the anterior surface of the natural lens in its non-accommodated state, measured by optical techniques. The anterior chamber depth, being the distance between the vertex of the cornea and the apex of the natural lens in rest and during accommodation, can be measured by means of ultrasonic equipment. Combining the values of the selected central radius and the estimated change in anterior chamber depth will result in the determination of the required minimal lens vault (see the definition in Fig. 2 for a definition of vault). The vault needs to be sufficiently large to avoid substantial intermittent contacts between the correction lens and the natural lens, but on the other hand the vault needs to be limited in order to avoid deformation or hindering of the iris. In order to fine tune the value of posterior radius, the radius of the anterior surface of the natural lens can be estimated in accommodated state so as to even more safely avoid significant intermittent contact outside the central part of the optic during accommodation. By estimating acceptable vault and posterior radius ranges, a specific lens with an acceptable vault and posterior central radius of a desired optical power can be selected.

In order to model the flawless curve as defined above different principles can be approached which are previously employed in optics for designing aspherical lenses, i.e. designing the anterior surface of a lens to reduce the spherical aberration (errors in refraction due to the spherical shape of a lens), for example in accordance with OSLO version 5 Program Reference, Chapter 4 (Update), Sinclair Optics 1996.

In a first aspect, the flawless curve comprises two or more tangentially attached circle segments. According to one embodiment of this aspect the flawless curve comprises three tangentially attached circle segments. For example, the three tangentially attached circle segments consist of a centrally located segment having a radius different to that of the natural lens in its non-accommodated state and two peripheral segments. In this specific example, the centrally located segment corresponds to the optical part and the peripheral segments correspond to the inner part of the support element. The three tangentially attached circle segments may thereby together form a fragment of an ellipsoidal curve.

In an other aspect, the flawless curve substantially follows the curve formula

$$z = cvr^2/(1 + \sqrt{(1 - cv^2(cc + 1)r^2)}),$$ where z is the axial coordinate of the curve, r is the radial coordinate of the curve, cv is the reciprocal central radius (1/rd where rd is the radius of curvature) of the optical part and cc is the conic constant to shape the curve which not is equal to zero. These curves are generally called conic curves. The conic constant can generally be selected according the following chart:

| | |
|---|---|
| CC = 0 | Sphere |
| CC = -1 | Paraboloid |
| CC <-1 | Hyperboloid |
| -1<CC<0 | Ellipsoid |
| CC>0 | Oblate Spheroid |

These curve designs are well known to optically skilled persons to model lenses having optics from corrected from spherical aberration. For example, conventional ocular lenses can be designed to deviate from a sphere in the peripheral region of their front (anterior) surfaces. However, in contrast to the present invention, the aspherical curve designs have been employed to model posterior surfaces of correction lenses. The design can be further optimized by adding one or several additional polynomal factors $a_1r^4 + a_2r^6 + a_3r^8 + a_4r^{10} + ... + a_nr^{2(n-1)}$, wherein $a_1$, $a_2$, $a_3$, $a_4$,....$a_n$ are aspheric constants, thereby generating the curve formula:

$$z = cvr^2/(1 + \sqrt{(1 - cv^2(cc + 1)r^2)}) + a_1r^4 + a_2r^6 + a_3r^8 + a_4r^{10} + ... + a_nr^{2(n-1)}$$

The selection of a posterior surface and a flawless curve design for a correction lens for the posterior chamber will in accordance with this aspect depend on the design of the optical part and the optical correction which has been determined as desirable for the patient. For example, if the optic is highly negative powered, i.e. more negative than about -15 diopters, the lens will have a convex-concave shape with a considerably thick edge profile (edge thickness) of the optical part which after implantation will consume a substantial volume of the available space in the posterior chamber of the eye. In such a case, the radius of the posterior surface proximal to the optical axis should be substantially smaller than radius of natural lens, i.e. less than about 8 mm, in order to avoid central adherence and to prevent deformation of the iris. A posterior surface can thereby be calculated and provided with a curve formula according to above, which will be substantially parabolic or hyperbolic. In another example, the lens is determined to have a positive power and the optic part will have a concave-concave shape with a central part proximal to the optical axis that will protrude anteriorly in the direction of the anterior chamber. The edge thickness of the optical part will be small and a posterior surface with a central radius larger than that of the natural lens is selected, i.e. larger than about 12 mm. The flawless curve representing the posterior surface will then be designed to bend towards the zonulas in the periphery and substantially follow an ellipsoidal curve formula.

In accordance with further aspects of the present invention, the flawless curve representing the posterior surface can also be constructed by other formulas and methods, e.g. by means of so-called Non uniform rational B-splines (NURBS), as referred to in I Piegl and W Tiller, The NURBS Book 2nd Ed, N.Y., Springer-Verlag 1997.

It is an important aspect of the present invention that the corrective lenses for the posterior chamber shall be freely floating in the aqueous humor of the posterior chamber and not having a permanent engagement with ciliary sulcus constituting its inner periphery. A free floating lens is consequently not kept in a constant position by the ciliary sulcus, but will to certain degree follow the eye movements, i.e. those of the natural lens during accommodation and the dilations of the pupil, while being surrounded by the aqueous humor flowing through the zonulas in anterior direction. For this reason, lenses according to the present invention, preferably will have a maximum diameter (including optic part and support means, i.e. haptic part) less than the average diameter of the ciliary sulcus. Suitably, the overall length of the lens (maximum diameter) is should not be less than about 1 mm than the ciliary sulcus to avoid excessive decentration of the lens from the optical axis. The overall lens length according to the invention is generally a compromise to obtain a floating effect and while retaining a centering effect from the sulcus. Therefor, the presently invented lenses will be centered by a combined controlled interaction with the iris and the ciliary sulcus. It is to be understood that the sulcus in practice is not circular, but rather elliptical and irregular, so a frequent touching contact of between the lens and the sulcus will in reality be attained which contributes to the mentioned centering effect. Should the correction lens not be sufficiently centered by the iris movements or the forces of the aqueous fluid between the correction lens and the natural lens, excessive decentration will prevented by the sulcus. For this reason and since the sulcus diameter has a tendency to shrink with increasing age of the patient, it cannot always be avoided that the overall length (maximum diameter) of the lens at least at some points exceeds the sulcus diameter. For lenses having a large diameter (above about 10.5 mm) the probability of sulcus contact increases considerably and thereby the risk of sulcus engagement that may lead to a compression of the lens and its axial displacement. However, by forming a peripheral part of the support means (haptic part) in accordance with the present invention this problem can be overcome for lenses having a larger diameter.

As earlier described, the support element comprise an inner part neighboring the optical part and a peripheral part which will at least partially to be in contact with the ciliary sulcus and the zonulas. The posterior surface of the support means have been earlier described so as to follow the flawless curve in a peripheral direction extending at least along the inner part.

At the anterior side, the inner part of the support element will comprise a tapered transition zone, surrounding the generally circular optical zone. The transition zone will extend from the edge of the optical part and smoothly decrease in crossectional thickness until a constant thickness of the inner part is reached.

According to a specific embodiment of the present invention, the peripheral part of the support means consists of two separate diametrically opposite, symmetrical parts. Preferably, each of the peripheral parts of the support means are provided with at least one peripherally located indentation of a generally concave shape extending inwards towards the inner part of the support means and the optical axis. Preferably, the indentation extends at its deepest point to the inner part of the support means and thereby generally divides each peripheral part into two identical sections which will at least partially be in contact with the ciliary sulcus, as earlier explained. From the mentioned indentations at least two diametrically opposite free spaces will after implantation be formed in the region between the peripheral parts and the ciliary sulcus wall. The preferred depth of the indentations is between about 0.5 to 1.25 mm. The indentations thereby form free spaces, which will both contribute to fluid circulation around the lens and to that the contact between the lens and the sulcus is restricted by these resilient peripheral members in a manner that the floating effect of the lens can be maintained, while the benefit of the contributory lens centering effect from the sulcus contact is retained.

The optical part of the correction lens is as mentioned above essentially circular and can be designed to correct various optical defects, including myopia and hyperopia. For example, the inventive correction lenses can be designed to correct astigmatism by designing their anterior surface toroidal or superimposing a cylindrical surface on the anterior side of the lens. As another example, the inventive lenses can correct presbyopia by applying a bi- or multifocal surface of the anterior side of the lens. The optically skilled person can readily apply a number of alternative anterior surfaces to provide a desired optical correction.

The size of optical part (the optical diameter) generally varies between about 4 to about 7 mm dependent on the patient and the desired optical correction. The presently invented correction lenses, having the aforedescribed posterior surfaces represented by a flawless curve, are more adapted to the available space for implantation of the posterior chamber. This provides that integrity of the surrounding eye tissues can be maintained and results in that a greater freedom of designing the optical part is admitted. It is of a particular advantage that larger optical parts having a diameter of at least about 5.5 mm can be selected also for optics with high refractive power which otherwise risk to bulky and negatively interfere with the natural lens. This will give the benefit of providing corrective lenses having an optical part substantially larger than the dilated pupil which results in less edge glare and undesired halo or cusp effects for the lens wearer when imaging strong light in darkness. It is therefore an important aspect of the invention to be able to provide lenses with reduced edge glare for high negative power lens having a refractive power less than about -15 diopters or high positive power lenses having a refractive power higher than about +15 diopters. Preferably, such high power lenses have an optical part larger than about 5.5 mm.

The present invention further relates to a method of selecting an intraocular correction lens adapted for the implantation in the posterior chamber of the eye. The method enables that a lens can be individually tailored for a patient and be manufactured prior to surgical intervention based on routine measurements of the eye. The selective method comprises the steps determining the power of optical correction needed for restoring the vision of the patient and estimating the anterior radius of the natural lens in its non-accommodated state. From this determination, a posterior central radius of the correction lens different to that of the natural lens in its non-accommodated state is selected and the total lens vault, as defined in the appended Fig. 2, is determined. A flawless curve free from points of inflection can thereby be designed or selected from a number of suitable design alternatives. As earlier defined, the flawless curve represents the intersection of the posterior surface and a plane containing the optical axis, so as to provide an aspheric posterior lens surface. The flawless curve will typically follow the alternatives outlined earlier and will provide a posterior surface that extends sufficiently beyond the extension of the natural lens so as to avoid the discussed drawbacks resulting from a built-up local pressure. In addition, the method can involve estimation of the anterior radius of the natural lens in its accommodated state and the estimation of the anterior chamber depth, preferably both in the accommodated and the non-accommodated states. From one or several of these values and the mentioned values of the anterior radius in its non-accommodated state and the posterior central radius selection, a total lens vault can be determined having a sufficient safety margin to avoid contacts between the natural lens and the posterior surface of the correction lens. Further measurements of the eye involves estimation of the diameter of the ciliary sulcus and an adaptation of the total lens diameter to this value. As earlier mentioned, the ciliary sulcus has an irregular shape varying between different individuals. For these purposes an average diameter value of the ciliary sulcus can serve as a basis for selecting suitable overall lens diameter and thereby considering that the correction lens preferably only should be in partial contact with the sulcus.

Based on the estimation of the necessary optical correction and above-mentioned eye measurements of the individual patients sufficient data can be transferred to the lens manufacturer so an individually adapted lens can be supplied. Alternatively, the surgeon can select the most suitable lens from a kit of pre-manufactured lenses with posterior aspherical surfaces according to the present invention by employing an algorithm. In such a selective method, it also conceivable to fine-tune the maximum diametric length of the correction to the individual ciliary sulcus estimation of the patient. This can be performed by a final corrective cutting of the lens before implantation by means of conventional mechanical tools or by means of an ophthalmic laser.

The kit contains lenses having a range of different optical powers with dimensional features resulting from an estimation of a suitable average population. In this case, it is to be understood that the surgeon is provided with an algorithm capable of transferring the physiological data to a suggested lens and from this result select the most appropriate lens present in the kit.

The lenses according to the present invnetion can be made from conventional biocompatible optically clear materials of a suitable refractive index by suitable molding technologies. Depending on the material, the lenses can be molded in one singular piece (silicones or poly(methyl)methacrylate (PMMA)) or be machined by precision milling and lathe cutting (PMMA or hydrogels). The lenses can be made from stiff materials like PMMA and similar acrylates. Alternatively, the lenses can be made of a material that is foldable or compressible like polysiloxanes, hydrogels such as polyHEMA, soft acrylates and the similar. A particularly suitable polysiloxane material is described in US Patent No. 5,306,297 and a particularly suitable hydrogel is described in US Patent No.5,717,049. The skilled person can readily conceive alternatives to these materials for the inventive correction lenses.

The corrective lenses will be described in more detail below according to a specific embodiment that serves to illustrate a non-limiting example of the present invention.

## Detailed description of the invention

Fig. 1A shows a definition of the cylindrical coordinate system.
Fig. 1B shows the cylindrical coordinate system applied on a correction lens implanted in the eye.
Fig. 2 shows a definition of the total lens vault.
Fig. 3A, 3B and 3C show posterior curve designs from circles or circle segments with discontinuities and inflection points.
Fig. 4 shows a paraboloid curve design
Fig. 5 shows the design of an oblate spheroid posterior surface.
Fig. 6 shows a surface constructed by spheres.

Fig. 7 shows a top view of an embodiment of the inventive corrective lenses
Fig. 8 shows a principal crossectional view along arrows A-A' in the embodiment of Fig. 1
Fig. 9 is detailed crossectional view of the peripheral part of the support elements

Fig 1 and Fig. 2 show the contemplated cylindrical coordinate system and its application in a lens implanted in the eye, wherein the z-axis coincides with the optical axis of lens.

The following example aims to demonstrate the design considerations when outlining a posterior surface according to the present invnetion. References are given to Fig. 2 to 6.

Example 1

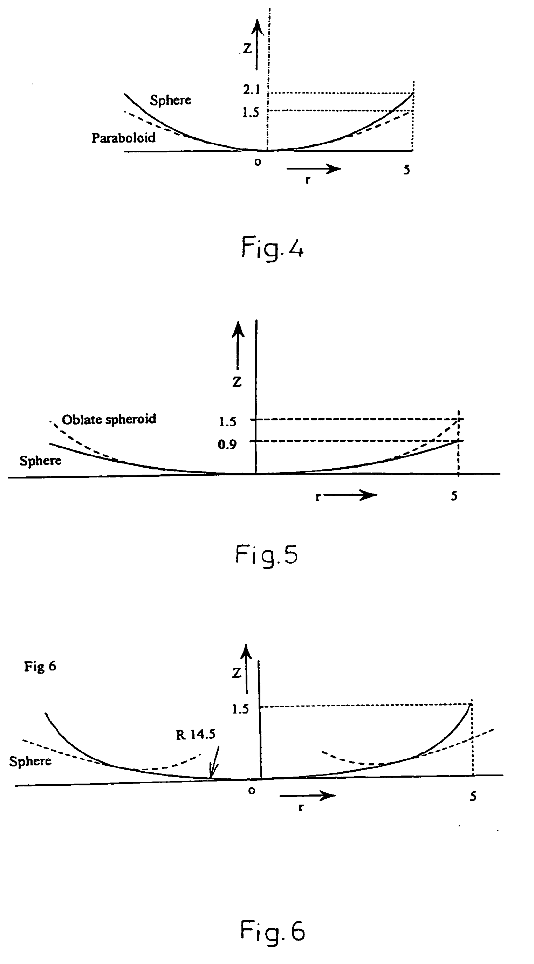

After estimation of the front radius of the natural lens of a patient, a 7 mm central posterior radius of the correction lens implant is selected. This selection enables that any adherence to the natural lens is avoided. In order to avoid any contacts with the natural lens, the correction lens should rest on the zonulas and vault over the natural lens. The vault is defined in accordance with Fig. 2. A vault of 1.5 mm is assumed to be sufficient to avoid contact, both with the accommodated and the non-accommodated natural lens. Should the posterior surfaces be spherical with a radius of 7 mm over 9 mm, the central vault of the correction lens is 2.1 mm as shown in Fig. 3A which is too large when considering the dimensions of the posterior chamber. In order to reduce the vault, the posterior surface is constructed from circle segments. The central circle segment thereby represents the posterior surface of the optical part and the peripheral segment represents the posterior surface of the inner part of the support means. As shown in Fig. 3B, the junction of the central segment to the peripheral segments shows a discontinuity. When the correction lens is pressed towards the crystalline lens by the iris, the posterior surface at this junction will be responsible for a circle shaped stress concentration at the surface of the natural crystalline lens with the effect that the central area between the natural lens and the correction lens will be sealed off from communication with fresh aqueous humor. The stress concentration can also result in damage of the anterior capsule of the natural lens. The transition zone between the two circles can be blended to obtain a smooth transition as demonstrated in Fig. 3C. This will reduce the potential stress concentration, but not entirely eliminate the risks of its appearance. The blended transition zone of Fig. 3C will decrease the optical zone or can change the refractive properties of the lens in an uncontrolled manner. In order to overcome these drawbacks a flawless posterior parabolic surface is designed as shown in Fig. 4. The central radius of surface is 7 mm and the surface is constructed as a conic surface following the curve:

$$z = cvr^2 / (1 + \sqrt{(1 - cv^2(cc + 1)r^2)})$$

having a conic constant and an inverted central radius of curvature cv = 0.143 mm$^{-1}$, resulting in a vault of 1.5 mm. The central part of the corrective implant still has optical properties if the anterior surface is spherical. This surface will vault sufficiently over the natural lens and it the implanted correction lens is pressed against the natural lens, there will be no risk of adherence due to the small radius and since the curve is flawless, there will be no stress concentrations.

Example 2

A large radius of curvature of 14.5 mm is selected to avoid adherence with the central radius of the natural lens. A spherical posterior surface is drafted with 10 mm in diameter, a radius of curvature of 14.5 mm and a central vault of 0.9 mm. To obtain a central vault of 1.5 mm, the surface is constructed as an oblate spheroid following the formula:

$$z = cvr^2 / (1 + \sqrt{(1 - cv^2(cc + 1)r^2)})$$

with a conic constant cc = 7.2. A rotation symmetric surface according to Fig. 5 is obtained with a 1.5 mm vault of the implant over a diameter of 10 mm. This surface will vault sufficiently over the natural lens and if the implanted lens will be pressed against the natural lens, there will be nor risk of adherence. Since the posterior surface will be flawless, the risk of stress concentration is eliminated. Fig. 6 shows an alternative design of a rotation symmetric posterior correction lens surface, according to which a curve is constructed in r-Z plane having a central circle segment with a radius of curvature of 14.5 mm is drafted. In the peripheries, circle segments with smaller radii of curvature are tangentially attached to the central segment to provide a flawless curve. A posterior surface having similar advantageous characteristics is thereby obtained.

Example 3

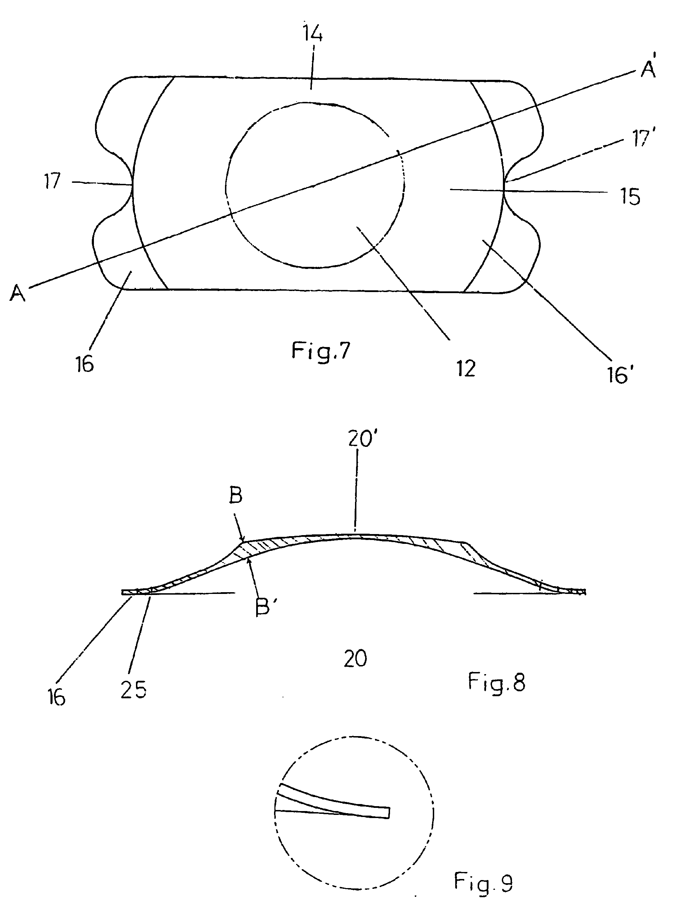

Fig. 7 shows a correction lens 10 lathe cut from a co-polymer of N-benzyl-N-methacrylamide with a refractive index of 1.49. The co-polymer is produced in accordance with US Patent No. 5,717,049. The lens has a maximum diameter of 12.5 mm and a central circular optical part 12 with a diameter of 6 mm and a support element 14. The support element consist of an inner part 15 surrounding the optical part and two peripheral parts 16 and 16' which after implantation will placed in the region of the ciliary sulcus of the eye and contribute to keep the lens in a central location. In this embodiment, the inner part has a diameter of 10 mm. The peripheral parts are each provided with a centrally located concave indentation 17, 17' that provide a free area for fluid circulation close to the ciliary sulcus.

Fig.8 shows a crossectional plane containing the optical axis along arrows A-A' in Fig.1. The intersection between this plane and the posterior surface of the lens represents a posterior curve that is arranged to be centrally flawless. As also demonstrated in Fig. 8, the optical part has a negative power of -12 diopters and an edge thickness shown long arrows B-B' of 0.45 mm. The maximum vault h of the lens is 1.7 mm. At a central position, in close proximity to the intersection with the optical axis 20-20', the radius of the posterior curve is 7 mm and thereby estimated to be substantially smaller than radius of the natural lens in its non-accommodated state. From the position close to optical axis, the posterior curve follows a parabolic curve having a curve formula

$$z = cvr^2 / (1 + \sqrt{(1 - cv^2(cc+1)r^2)})$$

with $cv = 0.143$ mm$^{-1}$ and $cc = -3$. The posterior flawless curve extends over the peripheral part of the optical part and along the posterior side of the supporting element to a point 25, which is a point of inflection. From this point, at which the inner part of the support means changes to the peripheral parts, the posterior surface is bended outwards away from the zonulas attaching the natural lens. The posterior curve of the peripheral part 16 is converging towards a plane perpendicular to the optical axis. The peripheral part is shown in detail by Fig. 9.

The lens according to Fig. 7 and 8, as described above, is designed to have reduced interference or disturbing contacts with the natural lens and may yet be designed with as large optical part as 6 mm and with a considerably low edge thickness. The posterior surface is free from areas or points that will create a local pressure on the natural lens when the correction lens moves in the posterior chamber during actions of self-centration when it touches the ciliary sulcus peripherally and the iris in an anterior direction. In addition, a parabolic posterior surface will enhance the centration of the lens due to its non-spherical design and its closer approximation of the natural lens when compared to an ellipsoid posterior surface. During accommodation, the shape of a young crystalline lens will change from a substantially spherical shape to a parabolic shape. At the same time, displaced aqueous fluid between the correction lens and the natural lens will exert a pressure both radial and axial pressure on the correction lens. The resulting force at a certain location of the correction lens will depend on the proximity to the natural lens. A close distance between the lenses will generate large forces on the correction lens and in this manner centering forces will be exerted on the correction lens during accommodation. For elder patients, who have lost their capability of accommodation, Thus, a lens with parabolic posterior surface, with a central radius larger than the central radius of the natural lens, can easily be centrated, as opposed to lenses with spherical or ellipsoid posterior surfaces.

**Claims**

1. An intraocular correction lens (10) for implantation in the posterior chamber of the eye between the iris and the intact natural lens comprising a centrally located optical part (12) capable of providing an optical correction and a peripherally located supporting element (14) capable of maintaining said optical part in said central location, **characterized in that** said optical part and said support element together have a concave posterior surface which is part of a non-spherical surface that is rotation symmetric around the optical axis of said optical part, wherein the intersection between said non-spherical surface and any plane containing the optical axis represents a flawless curve free from discontinuities and points of inflection.

2. A correction lens according to claim 1, wherein the flawless curve has substantially the same extension as the width of the lens.

3. A correction lens according to any of claims 1 or 2, wherein the peripheral part follows a curve diverging towards a plane perpendicular to the optical axis.

4. A correction lens according to claim 1, wherein the central radius of the posterior surface is less than about 7 mm.

5. A correction lens according to claim 1, wherein the central radius of the posterior surface is larger than about 14 mm.

**6.** A correction lens according to claim 1, wherein the radius of the posterior surface increases from the central part towards the lens periphery.

**7.** A correction lens according to claim 1, wherein the flawless curve comprises two or more tangentially attached circle segments.

**8.** A correction lens according to claim 7, wherein the flawless curve comprises three tangentially attached circle segments.

**9.** A correction lens according to claim 8, wherein the three tangentially attached circle segments consist of a centrally located segment having a radius different to that of the natural lens in its non-accommodated state and two peripheral segments.

**10.** A correction lens according to claim 9, wherein the centrally located segment corresponds to the optical part and the peripheral segments correspond to the inner part (15) of the support element.

**11.** A correction lens according to claim 10, wherein the three tangentially attached circle segments together approximate an ellipsoidal curve.

**12.** A correction lens according to claim 1, wherein the flawless curve substantially follows the curve formula
$$z = cvr^2 / (1 + \sqrt{(1 - cv^2(cc+1)r^2)})\,,$$
where z is the axial coordinate of the curve, r is the radial coordinate of the curve, cv is the reciprocal central radius of the optical part and cc is the conic constant to shape the curve which not is equal to zero.

**13.** A correction lens according to claim 12, wherein the curve formula is adjusted with one or several additional polynomal factors $a_1 r^4 + a_2 r^6 + a_3 r^8 + a_4 r^{10} + ... + a_n r^{2(n-1)}$, wherein $a_1, a_2, a_3, a_4, .... a_n$ are aspheric constants, thereby generating the curve formula:
$$z = cvr^2 / (1 + \sqrt{(1 - cv^2(cc+1)r^2)}) + a_1 r^4 + a_2 r^6 + a_3 r^8 + a_4 r^{10} +$$
$$... + a_n r^{2(n-1)}$$

**14.** A correction lens according to claim 1, wherein, the flawless curve representing the posterior surface is a spline polynome constructed from non-uniform rational B-splines (NURBS).

**15.** A correction lens according to claim 1, wherein the supporting element has a peripheral part and said supporting element consists of two separate diametrically opposite, symmetrical parts, (16, 16') each provided with at least one peripherally located indentation (17, 17') of a generally concave shape extending inwards towards the inner part of the support means and the optical axis.

**16.** A correction lens according to claim 15, wherein the indentation extends to the inner part of the support means.

**17.** A correction lens according to claim 16, wherein the indentation has a depth of about 0.5 to 1.25 mm.

**18.** A correction lens according to claim 1, wherein the flawless curve extends along the inner part of the supporting element.

**19.** A correction element according to claim 1, wherein the peripheral part of support element is provided with a higher flexibility than the inner part.

**20.** A correction lens according to claim 1, wherein the optical part has a diameter of a size sufficient to avoid edge glare.

**21.** A correction lens according to claim 20, wherein the optical part has a diameter of at least 5.5 mm.

**22.** A correction lens according to claim 20, having an optical power larger than $\pm 15$ diopters.

23. A method of selecting a suitable implantable correction lens according to any of claims 1 to 22, comprising the steps of:

(i) determining the power of optical correction;
(ii) estimating the anterior radius of the natural lens in its non-accommodated state ;
(iii) selecting a posterior central radius of the correction lens different to that of the natural lens in its non-accommodated state;
(iv) determining the total lens vault based on the data arriving from steps (ii) and (iii);
(v) selecting a flawless curve free from points of inflection representing the intersection of the posterior surface and a plane containing the optical axis so as to provide an aspheric posterior lens surface.

24. A method according to claim 23, comprising the step of measuring the anterior chamber depth and considering this value together with the data arriving from steps (ii) and (iii) in the determination of the total lens vault.

25. A method according to claim 24, wherein the anterior chamber depth is measured in both accommodated and non-accommodated states of the eye.

26. A method according to claim 23, further comprising the steps of estimating the length of ciliary sulcus and determining the maximum lens diameter.

27. A method according to claim 23, comprising the estimation of the anterior radius of the natural lens in its accommodated state and from this value in combination with the data arriving from steps (ii) and (iii) determining a maximum lens vault having a sufficient safety margin for avoiding contacts between the natural lens and the posterior surface of the correction lens.

28. A method of obtaining a suitable intraocular correction lens for implantation comprising the steps of:

(i) determining the power of optical correction;
(ii) estimating the anterior radius of the natural lens in its non-accommodated state;
(iii) selecting a posterior central radius of the correction lens different to that of the natural lens in its non-accommodated state;
(iv) determining the total lens height from the data arriving from steps (ii) and (iii);
(v) selecting a lens from a kit of correction lenses, wherein each lens have the features according to any of claims 1 to 22, said kit containing lenses with a range of different optical powers with dimensional features resulting from the estimation of a suitable average population.

29. A method according to claim 28, wherein said selection is based on employing an algorithm capable of transferring the physiological data to a suggested lens and from this result select the most appropriate lens present in the kit.

30. A method according to claim 28 comprising the step of measuring the anterior chamber depth and considering this value together with the data arriving from steps (ii) and (iii) in the determination of the total lens vault.

31. A method according to claim 30, wherein the anterior chamber depth is measured in both accommodated and non-accommodated states of the eye.

32. A method according to claim 28, comprising the estimation of the anterior radius of the natural lens in its accommodated state and from this value in combination with the data arriving from steps (ii) and (iii) determining a maximum lens vault having a sufficient safety margin for avoiding contacts between the natural lens and the posterior surface of the correction lens.

33. A method according to claim 28, further comprising the steps of estimating the length of ciliary sulcus and determining the maximum lens diameter.

34. A method according to claim 33, comprising the step of cutting the lens into a determined maximum diameter before implantation by means of mechanical tools.

35. A method according to claim 33, comprising the step of cutting the lens into a determined maximum diameter by means of an ophthalmic excimer laser.

**36.** A kit of intraocular lenses with a suitable variety of optical powers, wherein each individual lens is provided with the features according to any of claims 1 to 22.

**Patentansprüche**

**1.** Intraokulare Korrekturlinse (10) zur Implantation in die hinter Augenkammer zwischen die Iris und die intakte natürliche Linse, die einen zentral angeordneten optischen Teil (12) eingerichtet zum Bereitstellen von optischer Korrektur und ein peripher angeordnetes Halteelement (14) eingerichtet zum Festhalten des optischen Teils in der zentralen Position aufweist, **dadurch gekennzeichnet, dass** der optische Teil und das Halteelement zusammen eine konkave Hinterfläche aufweisen, die Teil einer nicht-sphärischen Fläche ist, die ihrerseits um die optische Achse des optischen Teils rotationssymmetrisch ist, wobei die Schnittmenge zwischen der nicht-sphärischen Fläche und einer beliebigen, die optische Achse aufweisenden Ebene eine fehlerfreie Kurve repräsentiert, die frei von Diskontinuitäten und Beugungspunkten ist.

**2.** Korrekturlinse gemäß Anspruch 1, wobei die fehlerfreie Kurve im Wesentlichen die gleiche Ausdehnung wie die Breite der Linse aufweist.

**3.** Korrekturlinse gemäß Anspruch 1 oder 2, wobei der Peripherteil einer Kurve folgt, die in Richtung einer Ebene senkrecht zur optischen Achse divergiert.

**4.** Korrekturlinse gemäß Anspruch 1, wobei der zentrale Radius der Hinterfläche kleiner als ungefähr 7 mm ist.

**5.** Korrekturlinse gemäß Anspruch 1, wobei der zentrale Radius der Hinterfläche größer als ungefähr 14 mm ist.

**6.** Korrekturlinse gemäß Anspruch 1, wobei der Radius der Hinterfläche von dem zentralen Teil in Richtung der Linsenperipherie zunimmt.

**7.** Korrekturlinse gemäß Anspruch 1, wobei die fehlerfreie Kurve zwei oder mehr tangential angebrachte Kreissegmente aufweist.

**8.** Korrekturlinse gemäß Anspruch 7, wobei die fehlerfreie Kurve drei tangential angebrachte Kreissegmente aufweist.

**9.** Korrekturlinse gemäß Anspruch 8, wobei die drei tangential angebrachten Kreissegmente ein zentral angeordnetes Segment mit einem Radius verschieden zu demjenigen der natürlichen Linse in ihrem unangepassten Zustand und zwei periphere Segmente aufweisen.

**10.** Korrekturlinse gemäß Anspruch 9, wobei das zentral angeordnete Segment dem optischen Teil entspricht und die peripheren Segmente dem Innenteil (15) des Halteelements entsprechen.

**11.** Korrekturlinse gemäß Anspruch 10, wobei sich die drei tangential angebrachten Kreissegmente zusammen einer ellipsoidischen Kurve nähern.

**12.** Korrekturlinse gemäß Anspruch 1, wobei die fehlerfreie Kurve im Wesentlichen der Kurvenformel

$$z = cvr^2 / (1 + \sqrt{(1 - cv^2(cc + 1)r^2)})$$

folgt, wobei z die axiale Koordinate der Kurve ist, r die radiale Komponente der Kurve ist, cv der reziproke zentrale Radius des optischen Teils ist und cc die konische Konstante zum Formen der Kurve ist, die ungleich Null ist.

**13.** Korrekturlinse gemäß Anspruch 12, wobei die Kurvenformel mit einem einzelnen oder mehreren zusätzlichen Polynom-Faktoren $a_1 r^4 + a_2 r^6 + a_3 r^8 + a_4 r^{10} + ... + a_n r^{2(n-1)}$ angepasst ist, wobei $a_1$, $a_2$, $a_3$, $a_4$ ... an asphärische Konstanten sind, wodurch die Kurvenformel erzeugt wird:

$$z = cvr^2 / (1 + \sqrt{(1 - cv^2(cc + 1)r^2)}) + a_1 r^4 + a_2 r^6 + a_3 r^8 + a_4 r^{10} + ... + a_n r^{2(n-1)}.$$

14. Korrekturlinse gemäß Anspruch 1, wobei die die fehlerfreie Kurve repräsentierende Hinterfläche ein Spline-Polynom ist, das aus ungleichförmigen rationalen B-Splines (NURBS) gebildet ist.

15. Korrekturlinse gemäß Anspruch 1, wobei das Halteelement ein Peripherteil aufweist und das Haltelement zwei separate, diametral gegenüber liegende symmetrische Teile (16, 16') ausweist, wovon jedes mit mindestens einer peripher angeordneten Vertiefung (17, 17') in einer im Allgemeinen konkaven Form bereitgestellt ist, die sich nach innen in Richtung des Innenteils des Haltemittels und der optischen Achse erstreckt.

16. Korrekturlinse gemäß Anspruch 15, wobei sich die Vertiefung zum Innenteil des Haltemittels erstreckt.

17. Korrekturlinse gemäß Anspruch 16, wobei die Vertiefung eine Tiefe von ungefähr 0,5 bis 1,25 mm aufweist.

18. Korrekturlinse gemäß Anspruch 1, wobei sich die fehlerfreie Kurve entlang des Innenteils des Halteelements erstreckt.

19. Korrekturlinse gemäß Anspruch 1, wobei der Peripherteil des Halteelements mit einer größeren Flexibilität als der Innenteil bereitgestellt ist.

20. Korrekturlinse gemäß Anspruch 1, wobei der optische Teil einen Durchmesser in einer Größe aufweist, die ausreicht, Kantenblendung zu vermeiden.

21. Korrekturlinse gemäß Anspruch 20, wobei der optische Teil einen Durchmesser von mindestens 5,5 mm aufweist.

22. Korrekturlinse gemäß Anspruch 20, welche eine optische Brennweite von größer als $\pm 15$ Dioptrien aufweist.

23. Verfahren zum Selektieren einer geeigneten implantierbaren Korrekturlinse gemäß einem der Ansprüche 1 bis 22, aufweisend die Schritte:

(i) Ermitteln der Brennweite der optischen Korrektur;
(ii) Abschätzen des Vorderradius der natürlichen Linse in ihrem unangepassten Zustand;
(iii) Auswählen eines zentralen Hinterradius der Korrekturlinse verschieden zu demjenigen der natürlichen Linse in ihrem unangepassten Zustand;
(iv) Ermitteln der gesamten Linsenwölbung basierend auf den von den Schritten (ii) und (iii) eintreffenden Daten;
(v) Auswählen einer fehlerfreien Kurve, die frei von Beugungspunkten ist und die Schnittmenge zwischen der Hinterfläche und einer die optische Achse aufweisenden Ebene repräsentiert, um auf diese Weise eine asphärische Linsen-Hinterfläche bereitzustellen.

24. Verfahren gemäß Anspruch 23, aufweisend die Schritte Messen der Vorderkammertiefe und Berücksichtigen dieses Wertes zusammen mit den von den Schritten (ii) und (iii) eintreffenden Daten bei der Ermittlung der gesamten Linsenwölbung.

25. Verfahren gemäß Anspruch 24, wobei die Vorderkammertiefe sowohl im angepassten Zustand des Auges als auch im unangepassten Zustand des Auges gemessen wird.

26. Verfahren gemäß Anspruch 23, außerdem aufweisend die Schritte Abschätzen der Länge des Ziliarsulkus und Ermitteln des maximalen Linsendurchmessers.

27. Verfahren gemäß Anspruch 23, aufweisend die Abschätzung des Vorderradius der natürlichen Linse in ihrem angepassten Zustand und von diesem Wert in Verbindung mit den von den Schritten (ii) und (iii) eintreffenden Daten Ermitteln einer maximalen Linsenwölbung, die einen ausreichenden Sicherheits-Spielraum zum Vermeiden von Kontakten zwischen der natürlichen Linse und der Hinterfläche der Korrekturlinse aufweist.

28. Verfahren zum Erhalten einer geeigneten intraokularen Korrekturlinse zum Implantieren, aufweisend die Schritte:

(i) Ermitteln der Brennweite der optischen Korrektur;
(ii) Abschätzen des Vorderradius der natürlichen Linse in ihrem unangepassten Zustand;
(iii) Auswählen eines zentralen Hinterradius der Korrekturlinse verschieden zu demjenigen der natürlichen Linse in ihrem unangepassten Zustand;

(iv) Ermitteln der gesamten Linsenhöhe aus den von den Schritten (ii) und (iii) eintreffenden Daten;

(v) Auswählen einer Linse aus einem Set von Korrekturlinsen, wobei jede Linse die Merkmale gemäß einem der Ansprüche 1 bis 22 aufweist, wobei das Set Linsen mit einem Bereich von verschiedenen Brennweiten mit dimensionalen Merkmalen, die aus der Abschätzung einer geeigneten durchschnittlichen Bevölkerung resultieren, aufweist.

29. Verfahren gemäß Anspruch 28, wobei die Auswahl auf einem Anwenden eines Algorithmus, der zum Übertragen der physiologischen Daten auf eine vorgeschlagene Linse eingerichtet ist, basiert und von diesem Ergebnis die am besten geeignete Linse, die in dem Set vorhanden ist, auswählt.

30. Verfahren gemäß Anspruch 28, aufweisend die Schritte Messen der Vorderkammertiefe und Berücksichtigen dieses Wertes zusammen mit den von den Schritten (ii) und (iii) eintreffenden Daten bei der Ermittlung der gesamten Linsenwölbung.

31. Verfahren gemäß Anspruch 30, wobei die Vorderkammertiefe sowohl im angepassten Zustand des Auges als auch im unangepassten Zustand des Auges gemessen wird.

32. Verfahren gemäß Anspruch 28, aufweisend die Abschätzung des Vorderradius der natürlichen Linse in ihrem angepassten Zustand und von diesem Wert in Verbindung mit den von den Schritten (ii) und (iii) eintreffenden Daten Ermitteln einer maximalen Linsenwölbung, die einen ausreichenden Sicherheits-Spielraum zum Vermeiden von Kontakten zwischen der natürlichen Linse und der Hinterfläche der Korrekturlinse aufweist.

33. Verfahren gemäß Anspruch 28, außerdem aufweisend die Schritte Abschätzen der Länge des Ziliarsulkus und Ermitteln des maximalen Linsendurchmessers.

34. Verfahren gemäß Anspruch 33, aufweisend den Schritt Schneiden der Linse in einen ermittelten maximalen Durchmesser vor der Implantation mittels mechanischer Werkzeuge.

35. Verfahren gemäß Anspruch 33, aufweisend den Schritt Schneiden der Linse in einen ermittelten maximalen Durchmesser mittels eines ophthalmischen Excimer-Lasers.

36. Set aus intraokularen Linsen mit einer geeigneten Vielzahl an Brennweiten, wobei jede individuelle Linse mit den Merkmalen gemäß einem der Ansprüche 1 bis 22 bereitgestellt ist.

**Revendications**

1. Lentille de correction intraoculaire (10) destinée à être implantée dans la chambre postérieure de l'oeil entre l'iris et le cristallin intact comprenant une partie optique située de façon centrale (12) capable d'assurer une correction optique et un élément de support situé de façon périphérique (14) capable de maintenir ladite partie optique dans ladite position centrale, **caractérisée en ce que** ladite partie optique et ledit élément de support ont ensemble une surface postérieure concave qui fait partie d'une surface non-sphérique qui est symétrique en rotation autour de l'axe optique de ladite partie optique, dans laquelle l'intersection entre ladite surface non-sphérique et n'importe quel plan contenant l'axe optique représente une courbe sans défaut exempte de discontinuités et de points d'inflexion.

2. Lentille de correction selon la revendication 1, dans laquelle la courbe sans défaut a sensiblement la même extension que la largeur de la lentille.

3. Lentille de correction selon l'une quelconque des revendications 1 ou 2, dans laquelle la partie périphérique suit une courbe divergeant vers un plan perpendiculaire à l'axe optique.

4. Lentille de correction selon la revendication 1, dans laquelle le rayon central de la surface postérieure est inférieur à 7 mm environ.

5. Lentille de correction selon la revendication 1, dans laquelle le rayon central de la surface postérieure est supérieur à 14 mm environ.

**6.** Lentille de correction selon la revendication 1, dans laquelle le rayon de la surface postérieure augmente depuis la partie centrale vers la périphérie de la lentille.

**7.** Lentille de correction selon la revendication 1, dans laquelle la courbe sans défaut comprend au moins deux segments de cercle attachés de façon tangentielle.

**8.** Lentille de correction selon la revendication 7, dans laquelle la courbe sans défaut comprend trois segments de cercle attachés de façon tangentielle.

**9.** Lentille de correction selon la revendication 8, dans laquelle les trois segments de cercle attachés de façon tangentielle consistent en un segment situé de façon centrale ayant un rayon différent de celui du cristallin dans son état non accommodé et deux segments périphériques.

**10.** Lentille de correction selon la revendication 9, dans laquelle le segment situé de façon centrale correspond à la partie optique et les segments périphériques correspondent à la partie interne (15) de l'élément de support.

**11.** Lentille de correction selon la revendication 10, dans laquelle les trois segments de cercle attachés de façon tangentielle approximent ensemble une courbe ellipsoïdale.

**12.** Lentille de correction selon la revendication 1, dans laquelle la courbe sans défaut suit sensiblement la formule de courbe $z = cvr^2/(1+\sqrt{(1-cv^2(cc+1)r^2)})$, où z est la coordonnée axiale de la courbe, r est la coordonnée radiale de la courbe, cv est le rayon central réciproque de la partie optique et cc est la constante conique pour former la courbe qui n'est pas égale à zéro.

**13.** Lentille de correction selon la revendication 12, dans laquelle la formule de courbe est ajustée avec un ou plusieurs facteurs polynomiaux supplémentaires $a_1r^4 + a_2r^6 + a_3r^8 + a_4r^{10} + ... + a_nr^{2(n-1)}$, où $a_1, a_2, a_3, a_4, ... a_n$ sont des constantes asphériques, générant ainsi la formule de courbe :

$$z = cvr^2/(1+\sqrt{(1-cv^2(cc+1)r^2)})+a_1r^4+a_2r^6+a_3r^8+a_4r^{10}+...+a_nr^{2(n-1)} \,.$$

**14.** Lentille de correction selon la revendication 1, dans laquelle la courbe sans défaut représentant la surface postérieure est un polynôme spline élaboré à partir de B-splines rationnelles non uniformes (NURBS)

**15.** Lentille de correction selon la revendication 1, dans laquelle l'élément de support possède une partie périphérique et ledit élément de support consiste en deux parties symétriques séparées diamétralement opposées (16, 16') dotée chacune d'au moins une indentation située de façon périphérique (17, 17') d'une forme généralement concave s'étendant vers l'intérieur vers la partie interne des moyens de support et l'axe optique.

**16.** Lentille de correction selon la revendication 15, dans laquelle l'indentation s'étend vers la partie interne des moyens de support.

**17.** Lentille de correction selon la revendication 16, dans laquelle l'indentation a une profondeur de 0,5 à 1,25 mm.

**18.** Lentille de correction selon la revendication 1, dans laquelle la courbe sans défaut s'étend le long de la partie interne de l'élément de support.

**19.** Elément de correction selon la revendication 1, dans lequel la partie périphérique de l'élément de support est dotée d'une plus grande souplesse que la partie interne.

**20.** Lentille de correction selon la revendication 1, dans laquelle la partie optique possède un diamètre d'une dimension suffisante pour éviter un éblouissement de bordure.

**21.** Lentille de correction selon la revendication 20, dans laquelle la partie optique a un diamètre d'au moins 5,5 mm.

**22.** Lentille de correction selon la revendication 20, ayant une puissance optique supérieure à $\pm$ 15 dioptries.

**23.** Procédé de sélection d'une lentille de correction implantable appropriée selon l'une quelconque des revendications 1 à 22, comprenant les étapes consistant à :

(i) déterminer la puissance de la correction optique ;
(ii) estimer le rayon antérieur du cristallin dans son état non accommodé ;
(iii) choisir un rayon central postérieur de la lentille de correction différent de celui du cristallin dans son état non accommodé ;
(iv) déterminer la voûte de lentille totale sur la base des données provenant des étapes (ii) et (iii) ;
(v) sélectionner une courbe sans défaut exempte de points d'inflexion représentant l'intersection de la surface postérieure et d'un plan contenant l'axe optique de façon à fournir une surface de lentille postérieure asphérique.

**24.** Procédé selon la revendication 23, comprenant l'étape consistant à mesurer la profondeur de la chambre antérieure et à considérer cette valeur conjointement avec les données provenant des étapes (ii) et (iii) dans la détermination de la voûte de lentille totale.

**25.** Procédé selon la revendication 24, dans lequel la profondeur de la chambre antérieure est mesurée à la fois dans les états accommodé et non accommodé de l'oeil.

**26.** Procédé selon la revendication 23, comprenant en outre les étapes consistant à estimer la longueur du sillon ciliaire et à déterminer le diamètre maximum de la lentille.

**27.** Procédé selon la revendication 23, comprenant l'estimation du rayon antérieur du cristallin dans son état accommodé et à partir de cette valeur en combinaison avec les données provenant des étapes (ii) et (iii) la détermination d'une voûte de lentille maximale ayant une marge de sécurité suffisante pour éviter les contacts entre le cristallin et la surface postérieure de la lentille de correction.

**28.** Procédé d'obtention d'une lentille de correction intraoculaire appropriée pour implantation comprenant les étapes consistant à :

(i) déterminer la puissance de la correction optique ;
(ii) estimer le rayon antérieur du cristallin dans son état non accommodé ;
(iii) sélectionner un rayon central postérieur de la lentille de correction différent de celui du cristallin dans son état non accommodé ;
(iv) déterminer la hauteur totale de la lentille à partir des données provenant des étapes (ii) et (iii) ;
(v) sélectionner une lentille dans un jeu de lentilles de correction, dans lequel chaque lentille a les caractéristiques selon l'une quelconque des revendications 1 à 22, ledit jeu contenant des lentilles ayant une plage de puissances optiques différentes avec des caractéristiques dimensionnelles résultant de l'estimation d'une population moyenne appropriée.

**29.** Procédé selon la revendication 28, dans lequel ladite sélection est basée sur l'emploi d'un algorithme capable de transférer les données physiologiques vers une lentille suggérée et de sélectionner, à partir de ce résultat, les lentilles les plus appropriées dans le jeu.

**30.** Procédé selon la revendication 28, comprenant l'étape consistant à mesurer la profondeur de la chambre antérieure et à considérer cette valeur conjointement avec les données provenant des étapes (ii) et (iii) dans la détermination de la voûte de lentille totale.

**31.** Procédé selon la revendication 30, dans lequel la profondeur de la chambre antérieure est mesurée à la fois dans les états accommodé et non accommodé de l'oeil.

**32.** Procédé selon la revendication 28, comprenant l'estimation du rayon antérieur du cristallin dans son état accommodé et à partir de cette valeur en combinaison avec les données provenant des étapes (ii) et (iii) la détermination d'une voûte de lentille maximum ayant une marge de sécurité suffisante pour éviter les contacts entre le cristallin et la surface postérieure de la lentille de correction.

**33.** Procédé selon la revendication 28, comprenant en outre les étapes d'estimation de la longueur du sillon ciliaire et de détermination du diamètre maximum de la lentille.

34. Procédé selon la revendication 33, comprenant l'étape de découpe de la lentille à un diamètre maximum déterminé avant l'implantation au moyen d'outils mécaniques.

35. Procédé selon la revendication 33, comprenant l'étape de découpe de la lentille à un diamètre maximum déterminé au moyen d'un laser à excimères ophtalmique.

36. Jeu de lentilles intraoculaires ayant une variété appropriée de puissances optiques, dans lequel chaque lentille individuelle est dotée des caractéristiques selon l'une quelconque des revendications 1 à 22.

Fig.1A

Fig.1B

Diameter Inner Part

Vault

r

o

z

Fig. 2

z

2.1

R 7

o

r

5

Fig. 3A

z

R peripheral
segment

R 7

1.5

o

r

5

Fig. 3B

z

R 7

1.5

R blend

o

r

5

Fig. 3C

Fig. 4

Fig. 5

Fig. 6

14

A'

17

17'

15

A

16

Fig.7

12

16'

20'

B

B'

16    25

20

Fig.8

Fig.9